# EUROPÄISCHE PATENTSCHRIFT

(11) **EP 0 826 005 B1**
(45) Veröffentlichungstag und Bekanntmachung des Hinweises auf die Patenterteilung: **12.05.2004**
(21) Anmeldenummer: 96920751.3
(22) Anmeldetag: 12.03.1996
(51) Int. Cl.: C07K 16/10, C07K 16/02, A23L 1/32

(54) **VERWENDUNG VON AVIÄREN, VITELLINEN, GEGEN HIV-ANTIGENE GERICHTETEN ANTIKÖRPERN ZUR HERSTELLUNG PHARMAZEUTISCHER ZUBEREITUNGEN**
USE OF AVIAN, VITELLIN ANTIBODIES DIRECTED AGAINST HIV ANTIGENS FOR THE MANUFACTURE OF PHARMACEUTICAL PREPARATIONS
UTILISATION D'ANTICORPS AVIAIRES, VITELLINS DIRIGES CONTRE LES ANTIGENES DU VIH POUR LA FABRICATION DE PREPARATIONS PHAMACEUTIQUES

(43) Veröffentlichungstag der Anmeldung: 04.03.1998
(73) Patentinhaber: Ovogenix Immunpharma GmbH, 13355 Berlin (DE)
(72) Erfinder: TSOLKAS, Panagiotis, D-21335 Lüneburg (DE); KOBILKE, Hartmut, D-14797 Damsdorf (DE)
(74) Vertreter: Wehlan, Helmut, Dr.
(86) Internationale Anmeldenummer: PCT/EP1996/000497
(87) Internationale Veröffentlichungsnummer: WO 1997/033915

(56) Entgegenhaltungen:
- EP-A- 0 152 270
- EP-A- 0 503 293
- DE-A- 19 504 755

## Beschreibung

Die Erfindung betrifft aviäre, vitelline, gegen HIV-Antigene gerichtete Antikörper, Verfahren zu ihrer Herstellung und ihre Verwendung. Die Antikörper - monospezifische, polyklonale Eidotter-Antikörper vom SPF-Huhn (Spezifiziert-Pathogen-Freien Huhn) - sind zur Diagnostik von HIV (Human Immunodeficiency Virus) und zur therapeutischen Anwendung bei HIV-positiven Patienten, im symptomfreien Stadium sowie bei Vorliegen der Symptome des ARC (AIDS-Related Complex) oder AIDS (Acquired Immuno Deficiency Syndrom), geeignet.

Es ist seit Ende des vorigen Jahrhunderts bekannt, daß im Eidotter des Hühnereis beträchtliche Mengen an Antikörpern (Ak) akkumuliert werden können (Klemperer F.: Arch. exptl. Pathol. Pharmakol. 31 (1893) 356-382). In den letzten zehn Jahren haben die Bemühungen stark zugenommen, Antikörper aus Vogeleiem zu gewinnen und medizinischen Zwecken nutzbar zu machen. Im Unterschied zu anderen Verfahren handelt es sich bei der Antikörper-Anreicherung und -Gewinnung via Vogelei um eine unblutige Methode, da die Blutentnahme für die Ak-Gewinnung entfallen kann (Schade, R. und A. Hlinak: Mh. Vet.-Med. 48 (1993) 91-98, Gustav Fischer Verlag Jena).

Die bekannten Verfahren bestehen darin, Hühner mit einem Antigen zu immunisieren und aus den Eiern / dem Eidotter die Antikörper (IgY - Yolk antibodies) zu gewinnen (US = USA-Patent, GB = Großbritannien-Patent, DD = DDR-Anmeldung, DE = Deutsche Offenlegungsschrift (OS) / Patentschrift (PS), EP = European Patent, WO = PCT (Patent Cooperation Treaty)-Anmeldung):
Immunologisch reaktive Präparate (Eidotter-Antikörper / Polson, A.: DE 2951412), Egg Yolk Antibodies (Polson, A.: GB 2057451, US 4357272), Fowl Egg Antibodies (Polson, A.: US 4550019), Specific Chicken Egg Antibodies (Tsuda, K. et al.: EP 503293), Specific antibody containing substance from eggs (Tokoro, H.: US 5080895, EP 225254).

Auch die passive Immunisierung von Säugern unter Verwendung von Antikörpern, die aus einer Geflügelart gewonnen wurden, ist beschrieben worden (Stolle, R. und L.R. Beck: DE 3504221, US 4748018, EP 152270). Demnach werden dem Säuger immunisierende Mengen eines Antikörpers verabreicht, der aus dem Ei einer Geflügelart gewonnen wurde, die mit dem die Krankheit des Säugers verursachenden Antigen immunisiert worden war.

Bekannt ist z.B. auch die erfolgreiche passive Immunisierung gegen Rota-Virusinfektionen bei Säugern mittels aviärer, vitelliner Antikörper (Bartz, C.R. et al., The Journal of Infectious Diseases, Vol. 142, No. 3, 1980, 439-441; Yolken, R.H. et al., Pediatrics (1988) 81 (2) 291-295; Kuroki, M. et al., Veterinary Microbiology, 37 (1993) 135-146); ebenso die passive Immunsierung von Säugern mittels aviärer Antikörper gegen E.coli (Wiedemann, V. et al., Journal of Veterinary Medicine, Series B (1990) 37 (3) 163-172; ebenso (1991) 38 (4) 283-291; Yokoyama, H. et al., Infection and Immunity (1992) 60 (3) 998-1007).

Die Strukturdifferenzen zwischen aviärem und Säuger-I_{g}G werden aus dem phylogenetischen Abstand von Vogel und Säuger erklärt. Sie werden vor allem im Unterschied der Molekulargewichte und der Sedimentationskonstanten - Hühner I_{g}G 170 000 bzw. 174 000, humanes I_{g}G 150 000 Dalton - gesehen (Larsson, A. und J. Sjöquist, Comp. Immun. Microbiol. infect. Dis., Vol.13, No.4, pp 199-201, 1990; Jürgens, L.: Reinigung von I_{g}G und I_{g}G-Antikörpern aus dem Eidotter, Dissertation 1987, Ludwig-Maximilians-Universität München).

Es ist ferner bekannt, daß HIV-infizierte Zellen auf ihre Zellmembran core-Antigene exprimieren, z.B. p24-Antigen (Nishino, Y. et al., Vaccine 1992, 10 (10) 677-683). Eine Hemmung der HIV-Infektion in vitro mittels anti-p24-Antikörpern beschreiben Gregersen,
J.P. et al. (J. Med. Virol. 1990, 30 (4) 287-293) und Franke, L. et al. (J. Med. Virol. 1992, 37 (2) 137-142). Der Einfluß eines hohen anti-HIVp24-Antikörper-Titers auf das Befinden HIV-positiver Patienten ist Gegenstand der WO 89/01339 (Cummins, L.M. et al.), der mit den Proteinen gp41 und p24 korrespondierenden Antikörper Gegenstand der WO 90/09805 (Zolla-Pazner, S. et al.). Die Beurteilung des immunologischen Abwehrprofils eines Patienten ist inhalt der DD 299090, der zufolge nach einer Behandlung mit monoklonalen Antikörpern (mAk), die insbesondere gegen das HIV-Protein p24 gerichtet sind (anti-HIV-p24-mAk), der anti-HIV-Ak-Gehalt des Patienten mit Hilfe eines ELISA (Enzyme Linked Immunosorbent Assay) bestimmt wird.

Mit dem massiven Abfall oder dem völligen Verschwinden der anti-p24-Antikorper aus dem Serum HIV-positiver Patienten setzt die Symptomatik des ARC beziehungsweise des AIDS ein (Jackson, G. G., THE LANCET, SEPT. 17. 1988, 647-651; Karpas, A. et al., Proc. Natl. Acad. Sci. USA, Vol. 87, pp 7613-7617, Oct. 1990), während Patienten mit hohem anti-p24-Antikörpertiter beschwerdefrei bleiben. Mehrere Autoren führten nun eine passive Immunisierung bei Patienten im Stadium des ARC und AIDS durch (Karpas, A., et al. 1990, ibid.; Karpas, A. et al., Int. Conf. AIDS, 1993 Jun 6-11, 9 (1): 244, abstract no. PO-A28-0659; Jackson, G.G., ibid.; Hague, R. et al., Int. Conf. AIDS, 1989 Jun 4-9, 5: 328, abstract no. T.B.P. 246; Lefrere, J. J. et al., a) Int. Conf. AIDS, 1993 Jun 6-11, 9 (1): 246, abstract no. PO-A28-0667; b) Rev. Fr.. Transfus. Hemobiol., 1991 May, 34 (3): 199-211; c) Int. Conf. AIDS, 1994 Aug 7-12, 10 (1): 226, abstract no. PB0335). Hierbei wurde Plasma von symptomfreien Patienten (bis 500 ml) mit hohem anti-p24-Antikörpertiter den Patienten im Stadium ARC und AIDS transfundiert, nachdem die Viren im Plasma durch Hitze inaktiviert waren. Zwei Stunden nach Transfusion war p24 im Serum nicht mehr nachweisbar, die Zahl der mit HIV infizierten Zellen reduzierte sich ebenfalls. Bei den Patienten im ARC-Stadium trat eine deutliche Besserung der klinischen Symptomatik bis zur Beschwerdefreiheit ein. Die Zahl der T4-Helferzellen fiel nicht weiter ab.

Ein Überleben bei Beschwerdefreiheit von bis jetzt schon 22 und 35 Monaten ist beschrieben worden. Bei Patienten im fortgeschrittenen Stadium von AIDS hielt die Remission 6 bis 22 Monate an, je nach Stand des individuellen Schweregrades der Erkrankung zum Zeitpunkt des Beginns der Behandlung, wobei eine Avirämie und ein ausreichender Antikörperspiegel bestand. Im Stadium des ARC dauerte die klinische Remission 22 Monate über die Studie KARPAS hinaus an, bei Avirämie und stabilen CD4+ T-Zellzahlen (Karpas, A. et al., Int. Conf. AIDS, 1993 Jun 6-11, 9 (1): 244, abstract no. PO-A28-0659; Bainbridge, D. et al., Int. Conf. AIDS, 1994 Aug. 7-12, 10 (1): 216, abstract no. PB 0293).

Der Erfindung liegt die Aufgabe zugrunde, gegen HIV-Antigene gerichtete Antikörper zur Verfügung zu stellen, die HIV-infizierten Patienten verabreicht werden. Die Aufgabe wurde dadurch gelöst, daß Hühner mit HIV-Antigenen immunisiert und dadurch anti-HIV-Antikörper im Eidotter angereichert werden. Überraschenderweise hat sich herausgestellt, daß die Gabe dieser Antikörper - in Form der oralen Verabreichung des getrockneten Eidotters oder als Antikörperextrakt nach Aufarbeitung und Isolierung aus dem Eidotter - zu erhöhten Antikörper-Serumtiterwerten führt und aviäre Antikörper im Säuger das Säugerkomplement zu binden vermögen.

Aufgrund des phylogenetischen Abstandes zwischen Vögeln und Säugern waren Geflügelarten für die Erzeugung von Antikörpern gegen Säugerkrankheiten ausgeschlossen worden, vor allem deshalb, weil das Hühnerprotein dem menschlichen Immunsystem fremd sei und bei wiederholter Verwendung zu allergischen Reaktionen führe (DE 3504 221 C2 vom 19.05.94) Auch zeigten Hühnerantikörper, so Gippner-Steppert et al., im Gegensatz zu den Säugetierantikörpem keine Reaktion mit Säuger-Komplementfactor C1 und Säuger-F_{c}-Rezeptoren (Gippner-Steppert, C. and M. Jochum: "Production and purification of chicken polyclonal anti-peptide antibodies specific for fibrino-elastase-peptide A-alpha1-21", Vortrag auf dem öffentlichen Jahresbericht-Colloquium der Chirurg.-Med. Klinik der LMU München am 04.02.1994).

Die Antikörper sind neu, sowohl als aviäre Antikörper als auch in ihrer Struktur. Sie unterscheiden sich von den bekannten Antikörpern durch Molekulargewicht, Sedimentationskonstante und isoelektrischen Punkt.

Erfindungsgemäß kommen insbesondere Antikörper zur Anwendung, die gegen HIV-core-Antigene gerichtet sind. Die wichtigste Rolle fällt dem Antikörper zu, der sich von dem core-Antigen p24 herleitet, gefolgt von den HIV-Proteinen p17, p7 (9), p12, p66 und p32 (Kageyama, S. et al., int. Conf. AIDS, 1994 Aug 7-12, 10 (2) : 86, abstract no PA 0224).

Nach bisherigem Wissen scheint der anti-p24-Antikörper HIV-positiver Patienten (HIV-1 und HIV-2) am effektivsten vor dem Auftreten der Stadien des ARC und AIDS zu schützen. Inwieweit andere Antikörper gegen core-Antigen eine zusätzliche Wirkung haben, ist noch nicht klar.
Zeitlich früher als der anti-p24-Antikörper fällt der Antikörpertiter gegen das core-Protein p17 ab (Lange, J.M. et al., AIDS, 1987 Sep, 1 (3) : 155-159).

Die anti-core-Antikörper - und insbesondere der Antikörper gegen p24-Antigen - bewirken eine weitgehende Beschwerdefreiheit und Lebensveriängerung bei Patienten im Stadium des ARC.

Die oben beschriebene Behandlungsmethode der Plasmatransfusion kann, schon aus logistischen Gründen, nur einem kleineren Kreis von Erkrankten zugute kommen.

Die aviären, vitellinen Antikörper gegen core-Proteine bieten den Vorteil, daß sie in großen Mengen vom Huhn produziert und an das Eidotter abgegeben werden. Erfindungsgemäß werden die Antikörper in hochgereinigter Form parenteral, aber auch oral als Trockeneigelb oder Antikörperextrakt verabreicht, da sie weitgehend hitzebeständig und säurefest sind. Mit der erfinderischen Lösung wird ein neues Nahrungsmittel angeboten: das anti-HIV-Antikörper enthaltende Hühnerei. Darin sind die Antikörper insbesondere, aber nicht ausschließlich, im Eidotter angereichert.
Als HIV-Antigene eignen sich insbesondere core-Antigene, vorzugsweise p24 und p17.
Sie führen, bei Verwendung von SPF-Hühnem, zu den entsprechenden erfindungsge-mäßen polyklonalen Antikörpern vom SPF-Huhn.
Das Verfahren zur Herstellung aviärer, vitelliner, gegen HIV-Antigene gerichtete Antikörper besteht darin, daß Hühner mit HIV-Antikörper, insbesondere mit core-Antigenen, vorzugsweise mit den core-Proteinen p24 und /oder p17, immunisiert, dadurch die Antikörper im Ei bzw. im Eidotter angereichert und gegebenenfalls daraus isoliert werden.
Die Antikörper lassen sich zur passiven Immunisierung von Säugern verwenden. Sie sind geeignet, HIV-positiven Patienten zur Erzeugung hoher Antikörper-Serumtiter verabreicht zu werden.
Die Isolierung der I_{g}Y-Antikörper erfolgt nach bekannten Verfahren aus wäßrigen Dotterlösungen mit PBS (phosphatgepufferter Kochsalzlösung) als Lösungsmittel und nachfolgender Zentrifugation, Ausfällung der Immunglobuline durch präzipitierende Agentien, wie Polyethylenglykol (PEG), Natrium-, Dextran- oder Ammoniumsulfat und / oder durch chromatographische Reinigung, wie Gelfiltration oder Ionenaustausch (Schade, R. und A. Hlinak: Mh. Vet.-Med. 48, 1993, S. 95, Gustav Fischer Verlag Jena).

Die Erfindung soll anhand von Ausführungsbeispielen näher erläutert werden.

### Ausführungsbeispiele

### Beispiel 1: Allgemeine Verfahrensweise

Die Immunisierung von Hühnern, insbesondere von SPF-Hühnem, wird mit synthetischem, nichtinfektiösen HIV-core-Vollantigenen und
a) Freund'schem kompletten Adjuvans (FCA)
b) Freund'schem inkompletten Adjuvans (ICFA)
vorgenommen.
Die Blut- und Eidotterprobenentnahme erfolgt an Pools von je 5 Hennen der Antigenversuchsgruppe sowie einer Negativ-Kontrollgruppe. Die Injektionsdosis beträgt 1,0 ml i.m. in die Brustmuskulatur in 4 Depots ä 0,5 ml resuspendiertes synthetisches Antigen und Adjuvans.
Beginn: mit Junghennen ab 15. Lebenswoche
Ende : 70. Lebenswoche (Legeende).
1.1. Erstimmunisierung (Basisimmunisierung)
mit 200 µg synthetischem HIV-core-Vollantigen (Protein) und FCA und Blut-Nullpro-
be am Tag "0" (Iₒ-Probe)
1.2. 1. Boosterung (2. Immunisierung)
   am Tag 28 mit Blut- und Eidotterprobe I₁ zur serologischen Antikörperkontrolle (I_{g}G / I_{g}Y), incl. Negativ-Kontrollgruppe.
   Alle Boosterungen erfolgen unter Verwendung von ICFA.
1.3. 2. Boosterung (3. immunisierung)
   am Tag 56 mit I₂-Blut- und Eidotterproben, incl. Neg.-Kontrollgruppe.
1.4. 3. Boosterung (4. Immunisierung)
   am Tag 84 mit I₃-Blut- und Eidotterproben, incl. Neg.-Kontrollgruppe.
1.5. I₄-Blut- und Eidotterproben
   am Tag 98, incl. Neg.-Kontrollgruppe.
1.6. I₅-Blut- und Eidotterproben
   am Tag 112, incl. Neg.-Kontrollgruppe.
1.7. I₆-Blut- und Eidotterproben
   am Tag 140, incl. Neg.-Kontrollgruppen.
1.8. I₇-Blut- und Eidotterproben
   am Tag 168, incl. Neg.-Kontrollgruppen.
1.9. 4. Boosterung (5.Immunisierung)
   am Tag 220 mit I₈-Blut- und Eidotterproben, incl. Neg.-Kontrollgruppen.
1.10. Eidotterseparierung mit Dottertrennung
   erfolgt nach dem BATCH-Verfahren bis zur Endreinigung und Extraktion der spezifischen HIV-Antikörper gegen die core-Vollantigene (Proteine) (Schade R. und A. Hlinak, Mh. Vet.-Med.48 1993,: S. 95, Gustav Fischer Verlag Jena): I_{g}Y-Präparation, Extraktion vitelliner Antikörper.
1.11. I_{g}Y-Bestimmung
   Die qualitativen und quantitativen Bestimmungen der spezifischen polyklonalen I_{g}Y-Eidotterantikörper, die im Durchschnitt 175 mg Antikörper / Eidotter betragen, erfolgen nach Schade R. und A. Hlinak (1993), ibid.
   Die quantitative Bestimmung erfolgt über spezifische Antikörper-Titer-Ermittlungen aus dem Dotterhomogenat mit Hilfe des indirekten ELISA, die des spezifischen Antikörpergehalts / Mengeneinheit Dotter über die Affinitätschromatographie (aus dem gereinigten und separierten Antikörperextrakt nach Vakuumdialyse).

### Beispiel 2: Verwendung von HIV-p24-Antigen

wie Beispiel 1, unter Verwendung von HIV-core-p24-Antigen.

### Beispiel 3: Verwendung von HIV-p17-Antigen

wie Beispiel 1, unter Verwendung von HIV-core-p17-Antigen.

### Beispiel 4: Isolierung der reinen Antikörper

erfolgt nach dem Batch-Verfahren (Schade, R. 1993, s. Beispiel 1.10) zur parenteralen und oralen Therapie.

## Patentansprüche

1. Verwendung von aviären, vitellinen, gegen HIV-Antigene gerichteten Antikörpern zur Herstellung von pharmazeutischen Zubereitungen, die zur passiven Immunisierung von Säugern geeignet sind.

2. Verwendung aviärer, vitelliner, gegen HIV-Antigene gerichteter Antikörper zur Herstellung von pharmazeutischen Zubereitungen, die HIV-positiven Patienten zur Erzeugung hoher Antikörper-Serumtiter verabreicht werden.

## Claims

1. Use of avian, vitelline antibodies directed against HIV antigens for the manufacture of pharmaceutical preparations which are suitable for passive immunisation of mammals.

2. Use of avian, vitelline antibodies directed against HIV antigens for the manufacture of pharmaceutical preparations which are administered to HIV-positive patients in order to produce high antibody serum titers.

## Revendications

1. Utilisation d'anticorps aviaires, vitellins, dirigés contre les antigènes du VIH pour la fabrication de préparations pharmaceutiques qui sont adaptées à l'immunisation passive de mammifères.

2. Utilisation d'anticorps aviaires, vitellins, dirigés contre les antigènes du VIH pour la fabrication de préparations pharmaceutiques qui sont données à des patients séropositifs pour la création d'un fort titre de sérum d'anticorps.
